# EUROPÄISCHE PATENTSCHRIFT

(11) **EP 2 253 312 B1**
(45) Veröffentlichungstag und Bekanntmachung des Hinweises auf die Patenterteilung: **15.04.2015**
(21) Anmeldenummer: 10008975.4
(22) Anmeldetag: 02.07.2004
(51) Int. Cl.: A61K 9/00, A61K 31/047, A61K 31/16, A61K 31/191, A61K 31/505, A61K 31/66, A61K 31/164, A61K 31/165

(54) **Verwendung von aus extremophilen Bakterien gewonnenen Osmolyten zur Herstellung von inhalierbaren Arzneimitteln zur Prophylaxe und Behandlung pulmonaler und kardiovaskulärer Erkrankungen, sowie eine Osmolyte als Wirkstoffbestandteil enthaltende Inhalationsvorrichtung**
Use of osmolytes obtained from extremophilic bacteria for the preparation of inhalable medicaments for the prophylaxis and treatment of pulmonary and cardiovascular diseases and an inhalation device containing osmolytes as active agents
Utilisation d'osmolytes obtenus à partir de bacteries extremophiles pour produire des médicaments inhalables pour la prophylaxie et le traitment de maladies pulmonaires et cardiovasculaires et un dispositif d'inhalation contenant des osmolytes comme composants actifs

(30) Priorität: 07.07.2003 DE 10330768
(43) Veröffentlichungstag der Anmeldung: 24.11.2010
(62) Teilanmeldung aus: 04740552.7
(73) Patentinhaber: Bitop AG, 58453 Witten (DE)
(72) Erfinder: Krutmann, Jean, Prof. Dr., 41844 Wegberg (DE)
(74) Vertreter: Thiel, Christian

(56) Entgegenhaltungen:
- WO-A-01/76572
- DATABASE BIOSIS [Online] BIOSCIENCES INFORMATION SERVICE, PHILADELPHIA, PA, US Juli 2009 SYDLIK ULRICH ET AL: 'The Compatible Solute Ectoine Protects against Nanoparticle-induced Neutrophilic Lung Inflammation' Database accession no. PREV200900428995 & AMERICAN JOURNAL OF RESPIRATORY AND CRITICAL CARE MEDICINE, vol. 180, no. 1, July 2009 (2009-07), pages 29-35, ISSN: 1073-449X, DOI: 10.1164/RCCM.200812-1911OC
- Abschlussbericht zum Projekt Wirkung kompatibler Solute auf nanopartikel-induzierte pathogene Endpunkte in der Lunge von Dr. Klaus Unfried, AG Partikel-Zell-Interaktion, Institut für Umweltmedizinische Forschung, Auf'm Hennekamp 50, 40225 Düsseldorf
- The influence of firoin on lung inflammation induced by carbon nanoparticles in rats, K. Unfried et al.
- The influence of different compatible solutes on apoptosis or proliferationinduced by carbon nanoparticles in lung epithelial cells, K. Unfried et al.

## Beschreibung

Die menschliche Gesundheit wird zunehmend durch Umweltnoxen beeinträchtigt. Besonders zu nennen ist hier die Luftverschmutzung mit Schwebstäuben, die faserförmiger oder partikulärer Natur sein können. In epidemiologischen Untersuchungen wurde gezeigt, dass Schwebstäube an der Entstehung von Lungenerkrankungen und kardiovaskulären Erkrankungen beteiligt sind. In großen europäischen Städten sind jährlich 60,000 Todesfälle die Folge einer langanhaltenden Luftverschmutzung. An dieser Luftverschmutzung sind Schwebstäube maßgeblich beteiligt. Es dürfte in absehbarer Zeit de facto unmöglich sein, die Belastung mit Schwebstäuben, insbesondere mit feinen und ultrafeinen Schwebstäuben durch Filtermassnahmen signifikant zu reduzieren. Es ist vielmehr mit einem erheblichen Ansteigen dieser Belastungen zu rechnen. Aus der URL *http:*//*propulmone.ch*/*Staubpartikel* ist es bekannt dass der Mensch im Laufe seines Lebens mindestems 400.000 m³ Luft einatmet Bei einer mittleren Partikelkonzentration in der Aussenluft von 30 µg/m³ und einem in der Lunge zurückgehaltenen Anteil von angenommen nur 20% werden pro Tag in jedem der 300 Millionen Alveolen etwa 100 Partikel abgelagert. In Raucherhaushalten ist die Belastung etwa 20 bis 45 % höher anzunehmen. Die gesundheitsschädlichen Wirkungen der Schwebstäube beruhen auf einer Interaktion dieser Mo-Ieküle mit dem menschlichen Lungengewebe. Die Folge davon sind entzündliche, zuweilen sogar maligne Lungenerkrankungen. In die Lunge gelangte staubförmige Fremdstoffe sind somit eine der wesentlichen Ursachen für die Entstehung von Lungenkrankheiten.

Außerdem wird angenommen, dass die Aufnahme von Schwebstäuben über die Lungenzellen und die in diesen Zellen in Folge ausgelösten biologischen Wirkungen auch für die Pathogenese kardiovaskulärer Erkrankungen zumindest mitverantwortlich sind.

Daraus folgt, dass die in der industriegesellschaft immer mehr zunehmende Belastung der Lunge durch Schwebstäube für eine nicht unbeträchtliche Erhöhung krankheitsbedingter Todesfälle verantwortlich gemacht werden muß
Aus obiger URL ist außerdem bekannt geworden, dass nach neueren Studien gesundheitliche Folgen sogar bei bisher als unbedenklich eingestuften Konzentrationen von Schwebstäuben anzunehmen sind. In verschiedenen Großstädten wurden Untersuchungen durchgeführt, die ergaben, dass mit der Erhöhung des Tageswertes der Staubbelastung um nur 10 µg/m³ eine Zunahme nicht unfallbedingter Todesfälle um 0,5 bis 1% einhergeht. Da, wie gesagt, die Konzentration an Schwebstaub in der Luft nicht wirklich gesenkt werden kann und der Gebrauch von Atemluft-Filtern auf Ausnahmefälle beschränkt bleiben muss, muss nach allgemein und einfach anwendbaren Mitteln gesucht werden, mit denen die Belastung der menschlichen Lunge vermindert und so die bekannten Folgeschäden möglichst gering gehalten werden können.
Es ist vor allem dringend geboten, für Menschen, die der täglichen Belastung mit Schwebstäuben z.B. am Arbeitsplatz nicht aus dem Weg gehen können, insbesondere für diejenigen, die an einer besondere Empfindlichkeit gegenüber Schwebstäuben leiden, vorbeugende Maßnahmen, zu entwickeln, die allgemein, einfach und jeder zeit eingesetzt werden können.

WO 01/76572 offenbart die orale oder topische Verwendung von Di-myoinositolphosphat, cyclischem 2,3-Diphosphoglycerat, 1,1-Di-glycerin-Phosphat, Firoin, Firoin-A, und/oder Di-mannosyl-di-inositolphosphat zum Schutz von Organismen vor Erkrankungen (Herz, respiratorischer Trakt etc.), die durch freie Radikale und oxidativ wirkenden Verbindungen ausgelöst werden.

Aufgabe der Erfindung ist es daher, pharmazeutische Mittel bereitzustellen, die im Stande sind, die oben beschriebenen negativen Auswirkungen von Schwebstäuben auf die Gesundheit des Menschen, insbesondere pulmonale und kardiovaskuläre Erkrankungen, wirksam zu bekämpfen.

Es wurde gefunden, dass Arzneimittel in inhalierbarer Form, welche als Wirkstoff einen oder mehrere Osmolyte, deren Salze und/oder gleichwirkende Derivate enthalten überraschend eine wirksame Prophylaxe gegen die oben beschriebenen Krankheitszustände und deren Behandlung ermöglichen.

Osmolyte werden aus extremophilen Bakterien gewonnen. Dies sind außergewöhnliche Mikroorganismen, die fähig sind, unter extremen Bedingungen, z.B. bei biologisch extremen Salzkonzentrationen bis 200 g Kochsalz pro Liter und bei Temperaturen im Bereich von 60 bis 110°C zu leben und sich zu vermehren. Solche Lebensbedingungen würden bei normalen (mesophilen) Organismen zum sofortigen Tod und mindestens zu massiven Schädigungen zellulärer Strukturen führen.
In den letzten Jahren wurde daher ein großer Forschungsaufwand betrieben, um diejenigen biochemischen Komponenten zu identifizieren, auf welche die bemerkenswerte thermische, chemische und physikalische Stabilität der Zellstrukturen extremophiler Organismen zurückzuführen ist.
Zu der hohen Temperaturstabilität von Zellstrukturen tragen in erheblichem Maß niedermolekulare organische Substanzen im intrazellulären Milieu bei, die als Osmolyte oder kompatible Solute bezeichnet werden. Die in den extremophilen Mikroorganismen gefundenen Osmolyte werden von menschlichen oder tierischen Zellen nicht gebildet.
In neuerer Zeit konnten in extremophilen Mikroorganismen erstmals verschiedene neuere Osmolyte identifiziert werden. Hierzu gehören beispielsweise Ectoin, Hydroxyectoin, Firoin, Firoin-A, Diglyceroiphosphat, cyclisches Diphosphoglycerat, Dimositolphosphat und 1,3-Di-mannosyl-di-myoinositolphosphat (DMIP).
Alle werden aus extremophilen Mikroorganismen gewonnen und aufgearbeitet bzw, gereinigt (vgl. EP-A 94 903 874; EP-A 98 121 243; DE-A 100 47 444) und bilden eine bekannte Gruppe niedermolekularer Stoffe mit Schutzeigenschaften für ansonsten sensible Zellen. In einigen Fällen konnte der Beitrag dieser Verbindungen zum Schutz von Hautzellen gegenüber externen Stressbedingungen wie Hitze- und Trokkenheit auf dem Gebiet der Kosmetik (vgl US-A 6 267 973,) gezeigt werden. Verschiedentlich wurde auch schon vorgeschlagen, topische Arzneimittel zum Schule der Haut vor externen Stresseinwirkungen oder zur Behandlung von Erkrankungen einzusetzen, die durch den enzymatischen Abbau von Gewebestrukturen verursacht werden (vgl. DE-A 100 06 578). Aufgeführt wurden neben anderen Krankheiten allgemein Erkrankungen des Immunsystems, Autoimmunerkrankungen, Entzundungsprozesse sowie akute und chronische Entzündungen.
Die ebenfalls auf die Verwendung von Osmolyten genchtete DE-A 198 34 816 betrifft Kosmetika mit einer Schutzwirkung gegen die UV-Bestrahlung der Haut, die daneben auch eine Wirksamkeit bei der Stabilisierung von Nukleinsäuren menschlicher Hautzellen aufweisen sollen Das Osmolyt Ectoin wurde auch als Feuchtigkeitsspender (moisturizer-) in kosmetischen Produkten eingesetzt, mit dem Ziel, die menschliche Haut gegen schädigende Wirkungen der ultravioletten Sonnenstrahlung zu schützen (EP-A 19 990 941)

Die Herstellung eines Arzneimittels zur allgemeinen Behandlung von Hauterkrankun gen mittels Osmolyten, insbesondere Ectoin oder Hydroxyectoin ist aus der EP-A 0 887 418 bekannt, die von der Anmelderin Bitop AG selbst hinterlegt wurde. Hierbei ging man davon aus, dass diese Wirkstoffe zur Stabilisierung von Enzymen und anderen Biomolekülen beitragen und daher zur Stabilisierung denaturierender Bedingungen beitragen können

in den Offenlegungsschriften DE-A 199 33 460, DE-A 199 33 461, DE-A 199 33 463 und DE-A 199 33 466 ist vorgeschlagen worden, Ectoine aufgrund einer antioxidativen Wirkung als Radikalfänger einzusetzen, und damit die Haut insbesondere vor der durch Sonnenbestrahlung beschleunigten und verstärkten Hautalterung zu schützen Damit sollten auch unerwünschte Zustände der Haut, die aus oxidativen Vorgängen resultieren, vermieden werden. Die WO 01/72287 beschreibt unter ähnlichen Prämissen wie In den zuletzt genannten Druckschriften vorgeschlagen, die Anwendung von Ectoinen bei der Behandlung von UV-induzierter Immunsuppression.

Bisher ist nicht bekannt, wie Osmolyte auf anderes Gewebe als das der Haut einwirken. Bereits die äußerliche Applikation von Hydroxyectoin auf Comea und Iris des Kaninchenauges (vgl. Heusener-Report No. T14952 v. 06.04.2001) verursachten anfangs Irritationen und Reizungen an der Bindehaut (Rötung, Chemose und Discharge), die zwar später nicht mehr beobachtet wurden, aber dem Fachmann einen klaren Hinweis auf zu erwartende Unverträglichkeiten bei empfindlicheren Gewebeoberflächen geben mußten. Angesichts dieser Erkenntnisse lag es für den Fachmann sicher nicht nahe, Osmolyte als zwar natürliche, aber schon aufgrund ihrer "unnatürlichen" Entstehung als körperfremde Wirkstoffe verdächtige Substanzen mit menschlichem Lungengewebe in Kontakt zu bringen. Eine Anwendung der Osmolyte am Gewebe im Körperinneren, insbesondere am äußerst rerzempfindlichen Lungen- oder Bronchialgewebe, wurde daher vom Fachmann bisher offenbar überhaupt nicht in Betracht gezogen.

Überraschenderweise hat sich nun aber gezeigt, dass Osmolyte vom menschlichen Bronchial- und Lungengewebe, einschließlich der Alveolen, nicht nur gut vertragen werden, sondern unerwartet auch eine hervorragende prophylaktische Wirkung gegen die schädlichen Einwirkungen von Schwebstäuben ausüben, unabhängig davon, welcher Natur die Schwebstäube sind. Sie eignen sich auch zur Behandlung von durch diese Einwirkungen kausal bedingten Erkrankungen.

Es ist somit möglich, durch eine geeignete Prophylaxe und/oder Behandlung mittels entsprechend dosierten Innalationsformen, welche Osmolyte als Wirkstoff enthalten, nicht nur die allgemein bekannten und oben beschriebenen Lungenerkrankungen, sondern auch die hieraus entstehenden kardiovaskulären Erkrankungen wirksam zu bekämpfen.

Gegenstand der Erfindung ist die Verwendung von Osmolyten, sowie von deren gleichwirkenden Derivaten und/oder pharmakologisch verträglichen Salzen bei der Bekämpfung von auf Schwebstaubeinwirkung auf das Lungengewebe beruhenden Krankheiten und/oder kausal hiermit verbundenen kardiovaskulären Erkrankungen

Ein weitere Gegenstand der Erfindung ist die Verwendung von Osmolyten wie in Anspruch 1 definiert, sowie von deren gleichwirkenden Derivaten pharmakologisch verträglichen Salzen, zur Herstellung von Arzneizubereitungen in inhalierbarer Form zur Anwendung in der Bekämpfung von auf Schwebstaubeinwirkung auf das Lungengewebe beruhenden Krankheiten und/oder hiermit verbundenen kardiovaskulären Erkrankungen.

Ein weiterer Gegenstand der Erfindung ist eine mit Wirkstoff befüllte Inhalationsvor richtung deren zerstäubbarer fester oder flüssiger Inhalt aus einer mindestens ein Osmolyt oder dessen Derivate und/oder pharmakologisch verträgliche Salze enthaltenden Wirkstoffzusammensetzung besteht.

Unter der Bekämpfung von Lungenkrankheiten wird folglich erfindungsgemäß sowohl die Prophylaxe bei gesunden Menschen, als auch die Behandlung von Menschen verstanden, bei denen die Symptome der Schwebstaubeinwirkung schon bestehen

Einige der erfindungsgemäßen Wirkstoffe sind schwache Basen oder Säuren und können daher auch, in manchen Fällen sogar bevorzugt, in ihrer pharmakologisch besonders verträglichen neutralen Salzform zum Einsatz kommen.

Als pharmakologisch verträgliche Salze kommen die Alkali- oder Erdalkalisalze, insbesondere die Salze des Kalium, Natrium, Magnesium und Calcium, aber auch Salzen mit organischen Basen wie z.B. mit nicht toxischen aliphatischen oder aromatischen Aminen in Frage.

Überwiegt im Falle der Anwesenheit von Stickstoffatomen im Wirkstoffmolekül die basische Natur, werden Salze mit pharmakologisch unbedenklichen organischen oder anorganischen Säuren, wie z.B. Essigsäure, Citronensäure, Weinsäure, Mandelsäure, Äpfelsäure, Milchsäure, Chlorwasserstaffsäure, Bromwasserstoffsäure, Schwefelsäure oder Phosphorsäure gebildet.

Gleichwirkende Derivate sind Verbindungen, die sich durch strukturelle Abweichungen insbesondere der funktionellen Gruppen und der Substituenten von den oben genannten Grundstrukturen der Osmolyten unterscheiden, aber im Sinne der Erfindung gleichwirkend sind. Im Falle des Hydroxyectoins können beispielsweise aus der Hydroxylgruppe mit gesättigten oder ungesättigten, geradkettigen oder verzweigten C¹ bis C⁴ Alkylgruppen entsprechende Alkoxylgruppen gebildet werden, Mit C¹ bis C⁴ Carbonsäuren werden entsprechende Ester gebildet. Aus der Carboxylgruppe entstehen Amide die wiederum gesättigte oder ungesättigte, geradkettige oder verzweigte C¹ bis C⁴ Alkylgruppen am Stickstoffatom aufweisen können. Mit entsprechenden C¹ bis C⁴ - Alkoholen erhält man wirksame Ester. Die Carboxylatgruppe wiederum kann durch eine Carbonyl- , Sulfonyl- oder Sulfonylatgruppe ersetzt werden. Entsprechende Modifikationen der übrigen genannten Osmolyte sind in analoger Weise unter Erhalt oder sogar Verbesserung der Wirkung möglich.

Allgemein können die erfindungsgemäßen Wirkstoffe oder deren Kombinationen ggf auch mit weiteren Wirkstoffen unter Einsatz von für die Inhalationstherapie üblichen und pharmakologisch unbedenklichen Hilfs- und Zusatzstoffen in bekannter Weise zu inhalierbaren Arzneimitteln verarbeitet werden.

Solche Zusätze sind bei inhalierbaren flüssigen Zubereitungen wegen der leichten Wasserlöslichkeit der Osmolyte in erster Linie steriles Wasser ggf. unter Zusatz von weiteren Lösungsmitteln, Stabilisatoren, Konservierungsmitteln oder Lösungsvermittlern

Als Aerosole werden Stoffsysteme bezeichnet, die aus festen und/oder flüssigen Partikeln bestehen, die fein in einem Gas vertellt sind. Im üblichen Sinn werden meist Wirkstoffe enthaltende Flüssigkeiten oft in Form von Lösungen zu Aerosolen versprüht.
Besonders praktisch in der Anwendung von Feststoffgemischen in sogenannten Pulverinhalatoren, mit denen diese Feststoffe für die Inhalation bereitgestellt werden können. Für die Verabreichung der Wirkstoffe stehen verschiedene Gerätetypen zur Verfügung die auf unterschiedlichen Sprühmechanismen beruhen, hierzu gehören z.B. Spinhaler, Diskhaler, Turbohaler, Rotahaler oder Aerolizer.

Gegenstand der Erfindung sind somit auch Medizinprodukte, die der erfindunhsgemäßen Anwendung dienen.

Erfindungsgemäß ist es grundsätzlich zu empfehlen, den Anteil an Hilfsstoffen im Inhalat auf ein Minimum zu begrenzen und für Pulverinhalatoren nur leicht resorbierbare nichtreizende Trägerstoffe wie z B. mikronisierte Laktose einzusetzen. Entsprechend mikronisierte Feststoffe sind als Trägersubstanzen besonders brauchbar, welche die Wirkstoffe in adsorbierter oder absorbierter Form enthalten, Diese Form der Inhalationstherapie hat sich in jüngerer Zeit mehr und mehr durchgesetzt, Die neueren Feststoffinhalatoren ermöglichen eine besonders einfache und sichere Applikation.

Es können neben den erfindungsgemäß vorgeschlagenen Wirkstoffen auch weitere für die Behandlung ggf. geeignete Wirkstoffe zugesetzt werden, Hierzu gehören beispielsweise Antiasthmatika, Broncholytika oder Expektorantia.

Flüssige Dosier-Aerosole können mit üblichen Treibmitteln, wie z B den FCKW-Treibmitteln Dichlor-difluor-methan, Tnchlor-fluormethan oder Cryofluoran verwendet werden Bevorzugt sind halogenfreie Treibmittel wie Propan oder Butan oder komprimierte untoxische Gase wie Stickstoff, Kohlendioxid oder Distickstoffmonoxid,

Die erfindungsgemäßen Wirkstoffe, können praktisch zu allen inhalierbaren Zubereitungsformen verarbeitet werden. Solche Zubereitungsformen sind beispielsweise Lösungen, Flüssig/Fest-Dispersionen, Fest/Fest-Dispersionen, Suspensionen und Emulsionen

Die Konzentration der Wirkstoffe liegt Im Bereich von 0,005 bis 20 Gew.% bezogen auf das Gewicht des eingesetzten Trägermaterials, Bevorzugt ist der Bereich von 0,05 bis 2 Gew.%.

Die folgenden Beispiele dienen zur Erläuterung der Erfindung, beschränken diese jedoch in keiner Weise.

### Beispiel 1

**Druckgas-Inhalationsmittel;**

| Inhaltstoff | Gehalt in Gewichts-% |
|---|---|
| Wasser | 97,0 |
| Ectoin | 0,5 |
| Hydroxyectoin | 0,5 |
| Konservierungsstoff | 0,2 |
| Treibgas Stickstoff | |

### Beispiel 2

**Pulver-Inhalatonsmittel:**

| Inhaltstoff | Gehalt in Gewichts-% |
|---|---|
| Lactone mikrokristallin | 99,4 |
| Ectoin | 0,3 |
| Hydroxyectoin | 0,3 |

## Patentansprüche

1. Arzneizubereitung zur Inhalation, enthaltend Firoin, Firoin-A, Diglycerolphosphat, cyclisches Diphosphoglycerat, 1,3-Dimannosyl-di-myo-inositol-phosphat (DMIP), Diinositolphosphat und/oder deren pharmakologisch verträgliche Salze, zur Anwendung in der Bekämpfung oder Prophylaxe von auf Schwebstaubeinwirkung auf das Lungengewebe beruhenden Krankheiten und/oder hiermit verbundenen kardiovaskulären Erkrankungen.

2. Arzneizubereitung zur Anwendung nach Anspruch 1, **dadurch gekennzeichnet, dass** der Zubereitung weitere Wirkstoffe zugesetzt sind.

3. Mit Wirkstoff befüllte Inhalationsvorrichtung, deren zerstäubbarer fester oder flüssiger Inhalt eine Wirkstoffzusammensetzung umfasst, die Firoin, Firoin-A, Diglycerolphosphat, cyclisches Diphosphoglycerat, 1,3-Dimannosyl-di-myo-inositol-phosphat (DMIP), Diinositolphosphat und/oder deren pharmakologisch verträgliche Salze enthält.

4. Inhalationsvorrichtung nach Anspruch 3 in Form eines gefüllten Inhalators für flüssige Arzneimittel.

5. Inhalationsvorrichtung nach Anspruch 3 in Form eines gefüllten Pulverinhalators.

6. Verwendung von Firoin, Firoin-A, Diglycerolphosphat, cyclischem Diphosphoglycerat, 1,3-Dimannosyl-di-myo-inositol-phosphat (DMIP), Diinositolphosphat und/oder deren pharmakologisch verträglichen Salzen zur Herstellung von inhalierbaren Arzneizubereitungen zur Bekämpfung oder Prophylaxe von auf Schwebstaubeinwirkung auf das Lungengewebe beruhenden Krankheiten und/oder hiermit verbundenen kardiovaskulären Erkrankungen.

7. Verwendung nach Anspruch 6, **dadurch gekennzeichnet, dass** der Zubereitung weitere Wirkstoffe zugesetzt werden.

## Claims

1. Pharmaceutical preparation for inhalation, comprising firoin, firoin-A, diglycerol phosphate, cyclic diphosphoglycerate, 1,3 dimannosyl di-myo-inositol phosphate (DMIP), diinositol phosphate and/or pharmacologically acceptable salts thereof for use in the treatment or prophylaxis of diseases caused by the effects of suspended particulate on the tissue of the lungs and/or the cardiovascular diseases that are related to them.

2. Pharmaceutical preparation for use according to claim 1, **characterized in that** further active agents are added to the preparation.

3. An inhalation device filled with active agent, the atomizable solid or liquid active agents contents of which comprising firoin, firoin-A, diglycerol phosphate, cyclic diphosphoglycerate, 1,3 dimannosyl di-myo-inositol phosphate (DMIP), diinositol phosphate and/or pharmacologically acceptable salts thereof.

4. Inhalation device according to claim 3 in the form of a filled inhaler for liquid pharmaceuticals.

5. Inhalation device according claim 3 in the form of a filled powder inhaler.

6. Use of firoin, firoin-A, diglycerol phosphate, cyclic diphosphoglycerate, 1,3 dimannosyl di-myo-inositol phosphate (DMIP), diinositol phosphate and/or pharmacologically acceptable salts thereof for the production of inhalable pharmaceutical preparations aimed at treatment or prophylaxis of diseases caused by the effects of suspended particulate on the lungs tissue and/or the cardiovascular diseases that are related with them.

7. Use according to claim 6, **characterized in that** further active agents are added to the preparation.

## Revendications

1. Préparation médicamenteuse pour inhalation, contenant de la firoïne, de la firoïne-A, du phosphate de diglycérol, du diphosphoglycérate cyclique, du phosphate de 1,3-dimannosyl-di-myo-inositol (DMIP), du phosphate de diinositol et/ou leurs sels pharmacologiquement acceptables, destinée à une utilisation pour la lutte contre les ou la prophylaxie des maladies dues à l'effet de particules en suspension sur le tissu pulmonaire et/ou des maladies cardiovasculaires associées.

2. Préparation médicamenteuse destinée à une utilisation selon la revendication 1, **caractérisée en ce que** des agents actifs supplémentaires sont ajoutés à la préparation.

3. Dispositif d'inhalation rempli avec un agent actif, dont le contenu solide ou liquide atomisable comprend une composition d'agents actifs, qui contient de la firoïne, de la firoïne-A, du phosphate de diglycérol, du diphosphoglycérate cyclique, du phosphate de 1,3-dimannosyl-di-myo-inositol (DMIP), du phosphate de diinositol et/ou leurs sels pharmacologiquement acceptables.

4. Dispositif d'inhalation selon la revendication 3, sous la forme d'un inhalateur rempli pour médicaments liquides.

5. Dispositif d'inhalation selon la revendication 3, sous la forme d'un inhalateur de poudre rempli.

6. Utilisation de firoïne, de firoïne-A, de phosphate de diglycerol, de diphosphoglycérate cyclique, de phosphate de 1,3-dimannosyl-di-myo-inositol (DMIP), de phosphate de diinositol et/ou leurs sels pharmacologiquement acceptables pour la fabrication de préparations médicamenteuses pouvant être inhalées pour la lutte contre les ou la prophylaxie des maladies dues à l'effet de particules en suspension sur le tissu pulmonaire et/ou des maladies cardiovasculaires associées.

7. Utilisation selon la revendication 6, **caractérisée en ce que** des agents actifs supplémentaires sont ajoutés à la préparation.
